# EUROPÄISCHE PATENTSCHRIFT

(11) **EP 0 863 995 B1**
(45) Veröffentlichungstag und Bekanntmachung des Hinweises auf die Patenterteilung: **21.05.2003**
(21) Anmeldenummer: 97942932.1
(22) Anmeldetag: 16.09.1997
(51) Int. Cl.: C12Q 1/44

(54) **VERBESSERTES VERFAHREN ZUR BESTIMMUNG VON LIPASE**
IMPROVED METHOD FOR DETERMINING LIPASE
AMELIORATION APPORTEE A UN PROCEDE POUR LA DETERMINATION DE LIPASE

(30) Priorität: 19.09.1996 DE 19638271; 01.08.1997 DE 19733309
(43) Veröffentlichungstag der Anmeldung: 16.09.1998
(73) Patentinhaber: Roche Diagnostics GmbH, 68298 Mannheim (DE)
(72) Erfinder: SCHELLONG, Lieselotte, D-82327 Tutzing (DE); ZIELENSKI, Ralf, D-83673 Bichl (DE); PRINZING, Urban, D-82380 Peissenberg (DE)
(86) Internationale Anmeldenummer: EP9705051
(87) Internationale Veröffentlichungsnummer: WO98012350

(56) Entgegenhaltungen:
- EP-A- 0 101 046
- EP-A- 0 207 252
- DE-A- 1 943 454
- US-A- 3 786 140
- DATABASE WPI Section Ch, Week 8602 Derwent Publications Ltd., London, GB; Class B04, AN 86-009992 XP002052402 & JP 60 233 560 A (FUJI REBIO KK) , 20.November 1985
- Y. OKAHATA & K. IJIRO: "Preparation of lipid-coated lipase and catalysis of glycerine ester syntheses in homogeneous organic solvents." BULLETIN OF THE CHEMICAL SOCIETY OF JAPAN, Bd. 65, Nr. 9, September 1992, TOKYO JP, Seiten 2411-2420, XP002052401

## Beschreibung

Die Erfindung betrifft ein Verfahren und Reagenz zur Bestimmung von Lipase in biologischen Flüssigkeiten sowie die Verwendung eines niedermolekularen N-substituierten Carbonsäureamid-Derivats zur Eliminierung von unspezifischen Reaktionen bei der Bestimmung von Enzymen. Insbesondere hat sich als vorteilhaft erwiesen, wenn N-(1,2-Dicarboxylethyl)-N-alkyl-sulfosuccinamid- oder N-(1.2-Dicarboxylethyl)-N-alkylaryl-sulfosuccinamid-Derivate in einer Konzentration von ca. 0.001 bis 2.0% (w/v) zugegen ist.

Die Bedeutung der Bestimmung der Lipase, insbesondere der humanen Pankreaslipase (E.C. 3.1.1.3.) für die Diagnostik und die Verlaufsbeurteilung von Pankreaserkrankungen ist seit längerem unumstritten (W. Steinberg et al.. Annals of Internal Medicine 102 (1985), 576: M. Panteghini et al.. Clin. Biochem. 24 (1991), 497; N.W. Tietz und D.F. Shuey, Clin. Chem. 39/5 (1993), 746). Beispielsweise kommt es bei akuter Pankreatitis innerhalb weniger Stunden zu einem, mitunter sehr massiven Anstieg der Lipase im Serum.

Die Aufgabe der Lipase im Körper besteht im wesentlichen darin, bevorzugt α-Esterbindungen von Triglyceriden mit langkettigen Fettsäureestern in einen Diglyceridanteil und die freie Fettsäure zu spalten. Anschließend erfolgt - allerdings erheblich langsamer - die Umsetzung zum Monoglycerid.

Üblicherweise verlaufen enzymkatalysierte Reaktionen in wäßriger Phase ab; die Lipase reagiert jedoch aufgrund ihrer physiologischen Bedeutung nur an der Grenzfläche Öl/Wasser. Hierdurch unterscheidet sie sich signifikant von den Esterasen. Außerdem sind Lipasen nur mit Hilfe der Colipase in der Lage, an eine durch Gallensäuren emulgierten Grenzfläche, ohne Denaturierung, anzulagern und Triglyceride zu spalten. Aus diesem Grund wird - neben den chemischen Parametern - die Reaktionskinetik maßgeblich durch die Qualität der dem Enzym angebotenen Grenzfläche beeinflußt.

Es sind heute unterschiedliche Verfahren zur Bestimmung von Lipase bekannt. Dabei handelt es sich im wesentlichen um titrimethische, turbidimetrische und immunologische Testprinzipien. Bei der titrimetrischen Bestimmung wird ein Überschuß von Lipasesubstrat. wie beispielsweise Olivenöl vorgelegt und die durch die Lipase daraus freigesetzte Menge an Fettsäure durch Titration mit Alkali unter Verwendung eines Indikators oder durch Extraktion der entsprechenden Kupfersalze gemessen. Titrimetrische Verfahren werden heute wegen der zum Teil schwierigen Handhabbarkeit, langen Reaktionsdauer und eines hohen Probenbedarfs nur noch selten in klinisch-chemischen Routinelabors eingesetzt (W. Junge in Methods of Enzymatic Analysis, Weinheim VCH. U. Bergmeyer ed., Vol. 4 (1984), 15).

Die turbidimetrische Lipasebestimmung, bei der die Trübunssaufhellung einer Trigiycerid/Wasser-Emulsion photometrisch verfolgt wird. ist heutzutage eine in klinisch-chemischen Routinelabors weitverbreitete Methode (W. Rick und M. Hockeborn. J. Clin. Chem. Biochem. 20 (1982), 735; J. Ziegenhorn et al., Medica Sonderheft 11 (1980)). Problematisch bei der Trübungsmessung ist. daß einzelne Serumproben keinen linearen Abfäll des Meßsignales innerhalb des Meßfensters des Photometers aufweisen oder sogar eine Trübungzunahme zeigen. Eine weitere Schwierigkeit dieser Bestimmungsmethode ist die reproduzierbare Herstellung einer Emulsion mit stets zuverlässiger gleicher Tröpfchengröße.

Immunologische Verfahren sind insbesondere mit dem Nachteil verbunden, daß bei diesem Prinzip die Masse und nicht die Enzymaktivität erfaßt wird (W. Uhl et al., Internat. J. Pancreatology 12/3 (1992), 253; G.E. Hoffmann et al., Ärztl. Lab. 30 (1984), 193; H. Herden und K. Walter, Klin. Lab. 38 (1992), 89).

Demzufolge wurden Testmethoden entwickelt und werden vornehmlich heute verwendet, die auf einer Farbentwicklung basieren. Beispielsweise werden 1.2-Diglyceride als Substrat verwendet, die durch Lipase zum 2-Monoglyceriden abgebaut und anschließend durch eine dem Test zugesetzte 2-Monoglyceridlipase zu Glycerin gespalten werden. Das auf diese Weise entstandene freie Glycerin wird mit Hilfe einer Glycerinphosphatoxidase zu Dihydroxyaceton und Wasserstoffperoxid (H₂O₂) abgebaut. Der Nachweis des entstehenden H₂O₂ erfolgt über ein entsprechendes Farbindikatorsystem (P. Fossati et al., Clin. Chem. 38/2 (1992), 211).

Auch die direkte Umsetzung eines Farbsubstrates ist heute bei der Lipase-Bestimmung möglich (EP 0 207 252). Die Aktivität der humanen Pankreaslipase setzt dabei direkt einen photometrisch bestimmbaren Farbstoff aus dem Substrat frei, wodurch eine aufwendige Enzymkaskade zur Produktion des Farbstoffes vermieden wird.

Ferner ist seit längerem bekannt, daß die Esterbindung der eingesetzten Farbsubstrate durch unspezifische Hydrolyse ein zu hohes Signal erzeugen. Beispielsweise ist problematisch. daß das Farbsubstrat para-Nitrophenylacetat durch Albumin und gamma-Globuline hydrolysiert wird (W. Downey und P. Andrews. Hiochem. J. 96 (1965) 21). Derartige Reaktionen ergeben eine Meßwertverfälschung, die insbesondere bei Serumproben zu einer klinisch falschen Aussage führen kann.

Der Erfindung liegt somit die Aufgabe zugrunde, unspezifische Reaktionen bei der Bestimmung von Lipase zu eliminieren und damit die Aussagekraft des Analysenergebnisses zu erhöhen.

Die Aufgabe wird gelöst durch ein Verfahren zur Bestimmung von Lipase durch Zugabe eines Lipase-Farbsubstrates und eines N-substituierten Carbonsäure-amid-Derivats mit einem Molekulargewicht bis zu 3000 Dalton mit anschließender photometrischer Bestimmung des aus dem Substrat freigesetzten Farbstoffes. Für das Carbonsäureamid-Derivat haben sich erfindungsgemäß insbesondere Verbindungen der allgemeinen Formel (I) bzw. (Ia) 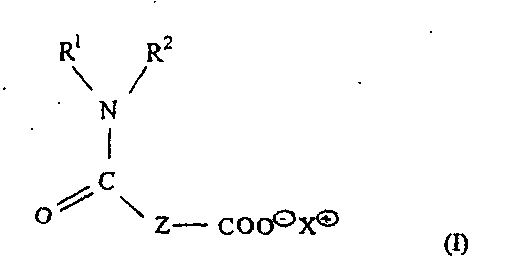 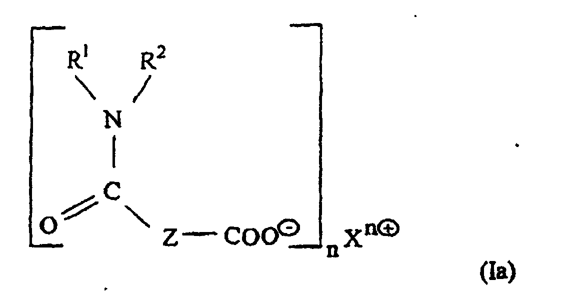 wobei R¹ und R², unabhängig voneinander, Wasserstoff oder einen gesättigten oder ungesättigten, substituierten oder unsubstituierten, gegebenenfalls carboxylierten Kohlenwasserstofftest mit 2 bis 24 Kohlenstoffatomen.

Z einen gesättigten oder ungesättigten, substituierten oder unsubstituierten, cyclischen oder geradkettigen Kohlenwassertoffrest mit 1 bis 10 Kohlenstoffatomen.

X ein Atom bzw. eine Atomgruppe mit positiver Ladung und n eine Zahl von 1 bis 3 darstellen, als geeignet erwiesen.

Bevorzugt sind erfindungsgemäß solche Carbonsäureamid-Verbindungen gemäß der allgemeinen Fromel (I) bzw. (Ia), wobei

Z eine Methylen- und/oder Ethylen-Gruppierung bestehend aus einem bis zehn C-Atomen und gegebenenfalls mit einer elektronenziehenden Gruppe wie eine Carboxyl-, Sulfonyl-, Phosphat-, Phosphonat-, Nitro-, Nitrit- oder Nitrat-, Halogen- oder Alkoxygruppe substituiert ist.

R¹ und R², unabhängig voneinander. Wasserstoff, eine gegebenenfalls substituierte Alkyl-, Aryl-, Alkylaryl- oder Alkylengruppe, geradkettig oder verzweigt, gesättigt oder ungesättigt, bestehend aus drei bis 24 C-Atomen, wobei ein Carboxyl- oder Dicarboxylalkylrest, betehend aus zwei bis 24 C-Atomen besonders bevorzugt ist. und

X ein Atom bzw. eine Atomgruppe mit positiver Ladung darstellt und n die Zahl 1 oder 2 bedeutet. als geeignet erwiesen.

Erfindungsgemäß kommen insbesondere solche Verbindungen in Betracht. worin der Rest Z eine Carboxyl- und/oder Sulfonylgruppe trägt oder auch solche, in denen einer der Reste R¹ oder R² eine niedrige Alkylgruppe, wie beispielsweise eine Methyl-, Ethyl-. Propyl-, Butyl, Pentyl-, eine Ethylenyl- oder eine Propylenylgruppe oder eine C10- bis C18-Alkylgruppe bedeutet. Die Substitution entsprechender Alkyl- bzw. Alkylengruppen erfolgt gegebenenfalls nach bekannten Methoden, Weiterhin hat sich als vorteilhaft erwiesen, wenn beispielsweise R¹ einen Dicarbonsäurerest auf Basis von Malonsäure, Glutarsäure, Adipinsäure, Pimelinsäure, Azelainsäure und Sebacinsäure und insbesondere Bernsteinsäure darstellt. Die erfindungsgemäß verwendbaren Verbindungen weisen vorzugsweise ein Molekulargewicht von ca. 200 bis 1000 Dalton auf; es haben sich darüber hinaus jedoch auch solche als geeignet erwiesen, die bis zu 3000 Dalton entsprechen. Als ganz besonders vorteilhaft haben sich Verbindungen bzw. Salze, die mindestens zwei Säuregruppen aufweisen, wie z.B. das Tetranatriumsalz auf Basis von N-(1,2-Dicarboxyethyl)-N-alkyl-sulfosuccinamid mit niedriger Alkylgruppe (1 bis 6 C-Atome) und/oder höherer Alkylgruppe (12 bis 18 C-Atome) oder N-(1,2-Dicarboxyethyl)-N-alkylaryl-sulfosuccinamide bzw, davon abgeleitete Derivate und darüber hinaus entsprechende Gemische erwiesen.

Prinzipiell können sämtliche Verbindungen, die von Lipase als Substrat erkannt und umgesetzt werden, für die Bestimmung von Lipase in Gegenwart einer der erfindungsgemäßen Verbindung, wie beispielsweise gemäß der allgemeinen Formel (I) bzw. (Ia), verwendet werden. Besonders vorteilhaft hat sich ein Verfahren zur Bestimmung von Lipase erwiesen, bei dem Verbindungen gemäß EP 0 207 252 als Lipasesubstrat, z.B. 1,2-O-Dioctylrac-glycero-3-azelainsäure-, 1,2-O-Didecyl-rac-glycero-3-pimelinsäure-, 1,2-O-Didodecylrac-glycero-3-glutarsäureresorufinester oder 1,2-O-Dilauryl-rac-glycero-3-glutarsäure-(4'oder 6'-methylresorufin)-ester, eingesetzt werden. Dabei hat sich überraschenderweise gezeigt, daß die erfindungsgemäßen Substanzen in der Lage sind, in einer Emulsion, die ein entsprechendes Farbsubstrat enthält, vor einer unspezifischen Hydrolyse zu schützen, ohne dabei gleichzeitig die Aktivität der Lipase zu hemmen.

Eine Verbindung gemäß der allgemeinen Formel (I) bzw. (Ia) mit den oben angegebenen Bedeutungen für R¹, R², Z, X und n oder ein Gemisch mehrerer erfindungsgemäßer Verbindungen wird der Reaktionslösung für die Lipase-Bestimmung in einer Konzentration (Endkonzentration) von ungefähr 0,001 bis 2,0% (w/v), vorteilhafterweise von ca. 0,01 bis 1,0% (w/v) zugesetzt.

Weitere Bedingungen bzw. Zusätze, wie beispielsweise Detergenzien, Emulgatoren (z.B. Taurodesoxychlorat, Natriumdesoxycholat, Brij 35, Triton X-114), bakterizid oder fungizid wirkende Substanzen (Natriumazid, Methyl-iso-thiazolon u.a.), Stabilisatoren (z.B. Dilaurylglycerol), Cofaktoren. Aktivatoren oder Maßnahmen zur Vermeidung ungewünschter (Neben)reaktionen, für die Lipase-Bestimmung sind dem Fachmann bekannt. Insbesondere haben sich hier die in DE 29 04 305 oder EP 0 207 252 beschriebenen Zusätze und Spezifikationen als geeignet erwiesen. Die Bestimmung der Lipase kann sowohl in Proben menschlichen, als auch tierischen Ursprungs (z.B. Schweinepankreaslipase), wie z.B. Blut, Serum oder Gewebe erfolgen.

Die Herstellung der erfindungsgemäßen N-substituierten Carbonsäureamid-Verbindungen bzw. Salze insbesondere gemäß der allgemeinen Formel (I) bzw. (Ia), erfolgt nach dem Fachmann bekannten Methoden. Darüber hinaus können die erfindungsgemäßen Verbindungen, wie beispielsweise Tetranatrium-N-(1,2-dicarboxylethyl)-N-alkylsulfosuccinamid (REWOPOL B 2003) von einschlägigen Firmen, wie z.B. der Firma Witco Surfactants GmbH, Steinau, kommerziell bezogen werden.

Ein weiterer Gegenstand der Erfindung ist ein Reagenz bzw. Reagenzkit zur Bestimmung von Lipase, das/der im wesentlichen aus folgenden Komponenten besteht: (a) einem Lipese-Farbsubstrat, (b) einer geeigneten Puffersubstanz sowie (c) - enthalten in einem beliebigen Teilreagenz - eines N-substituierten Carbonsäureamid-Derivats, mit einem Molekulargewicht bis zu 3000 Dalton d.h. beispielsweise einer Verbindung gemäß der allgemeinen Formel (I) bzw. (Ia). Als Puffersubstanz eignen sich alle bekannten Puffer, welche in der Lage sind, im Rahmen des er-5 findungsgemäßen Reagenzes einen pH-Wert zwischen 6,0 und 10,5 einzustellen. Der bevorzugte pH-Wertbereich liegt zwischen 7,0 und 9,5. Beispiele für geeignete Puffer sind Diethanolaminpuffer, Triethanolamiapuffer, Tris- oder Tartrat-Puffer oder sogenannte Good'sche Puffer, wie Hepes-Puffer, Bicine-, Taps-Puffer, CHES-Puffer (2-(Cyclohexylamino)-ethansulfonsäure-Puffer) und Tris. Besonders bevorzugt wird Bicine-Puffer. Die Pufferkonzentration liegt hier üblicherweise zwischen 5 und 200 mM, bevorzugt zwischen 30 und 100 mM, ganz besonders bevorzugt bei ca. 50 mM.

Als vorteilhaft hat sich erwiesen, wenn das Reagenz aus zwei Reagenzkomponenten besteht, wobei neben weiteren, dem Fachmann bekannten Zusätzen das eifindungengemäße Carbonsäureamid-Derivat im ersten und das Lipasefarbsubstrat im zweiten Teilreagenz enthalten ist. Wesentlich ist dabei, daß - vor der Bestimmung - die erfindungsgemäße Carbonsäureamidverbindung im leicht alkalisch gepufferten Medium und das Lipasefarbsubstrat bei saurem pH-Wert gehalten werden. Eine besondere Ausführungsform des erfindungsgemäßen Reagenzes besteht aus zwei Teilreagenzien, wobei das erste einen im schwach Alkalischén gepufferten pH-Wert (pH 7,5 bis 9,0, bevorzugt ca. pH 8,0) aufweist, sowie ein oder mehrere erfindungsgemäße Carbonsäureamid-Verbindungen der allgemeinen Formel (I) oder (Ia) und weitere Salze, Konservierungsmittel sowie Detergenzien beinhaltet. Das zweite Teilreagenz enthält im wesentlichen eine im Bereich von pH 2,0 bis 5,0, bevorzugt ca. pH 4.0 puffernde Substanz, ein geeignetes Lipasesubstrat sowie gegebenenfalls weitere Hilfsstoffe. Als besonders vorteilhart hat sich für ein solches zweites Teilreagenz erwiesen, wenn unter Druck mittels Einspritztechnik geeignete Emulgatoren, wie beispielsweise Cholatverbindungen und/oder Thesite, beigemengt werden. Bevorzugt wird hierfür die Ölphase, d.h. das sämtliche Komponenten aufweisende Reagenz R2, durch eine sehr feine Kanüle in eine vorgelegte wäßrige Phase eingespritzt. Im Ergebnis wird durch die beschriebene Maßnahme eine klare, nicht-trübe Emulsion/Suspension verbesserter Stabilität erhalten. Darüber hinaus ist das Reagenz zu keinen unerwünschten Nebeoreaktionen - beispielsweise aufgrund der Gegenwart von unspezifischen Enzymen (nicht-Lipase-Esterasen) - befähigt. Ein solches, durch eine einheitliche Partikelgröße gekennzeichnetes Lipasesubstrat-Reagenz kann bei ca. 2 bis 8°C ohne nennenswerten Qualitätsverlust zwischen 12 und 18 Monate gelagert werden. Dadurch wird zudem eine entsprechende Stabilität des Gesamtreagenzes gewährleistet. Das Maximum der Größenverteilung der Partikel liegt vorteilhafterweise bei ca. 100 nm, was in diesem Fall einer Trübung von weniger als 300 NTU (Nephelometric Turbidimetric Unit) entspricht.

Erläuterung der Abbildungen:
Abbildung 1: IgG-Abstufüne 0 - 3 g/dl: R1 (Reagenz 1) ohne Zusatz erfindungsgemäßer Verbindung: Probe: 5 µl;
   ― LW NaCl: --- IgG 0: ······ IgG 0.5 g/dl; -·-·-·^{.} IgG 1.0 g/dl; -··-·· IgG 1.5
   g/dl; ― IgG 2.0 g/dl; --- IgG 2.5 g/dl; ······ IgG 3.0 g/dl.
Abbildung 2: IgG-Abstufung 0 - 3 g/dl; R1 mit 0.27 % (w/v) Tetranatrium-N-(1,2-Dicarboxyethyl)-N-alkyl-sulfosuccinamid: Probe: 10 µl; übrige Angaben wie in Legende zu Abbildung 1.
Abbildung 3: Lipase, turbidimetrisch (Reagenz 1, R1), und Farbtest (Reagenz, R2); R1/R2 ohne Zusatz erfindungsgemäßer Verbindung: Kalibrator: Cfas (Calibrator for automated Systems)
   Anzahl der Wertepaare: 25
   ― BaPa y = 57.8108 + 1.0315*x (r = 0.9828)
   -^{.}-^{.}- W.Halb y = x
   x Humanseren
Abbildung 4: Lipase, turbidimetrisch (R1), und Farbtest (R2); R1 ohne, R2 mit erfindungsgemäßer Verbindung wie in Abbildung 2: sonstige Bedingungen siehe Legende zu Abbildung 3.
   Anzahl der Wertepaare: 25
   ― BaPa y = 12.6916 + 1.3995*x (r = 0,9944)
   ······ Std. Hk y = 9.4818 + 1.4178*x
   -·-·- W.Halb y = x
   x Humanseren

Die folgenden Beispiele erläutern die Erfindung weiter:

### Beispiel 1

Verschiedene Konzentrationen von IgG als Probe

Reagenzzusammensetzung Lipase Farbtest:

| | | |
|---|---|---|
| Reagenz 1 (R1): | 4,55 mmol/l | Taurodesoxycholat |
| | 1,77 mmol/l | Na-Desoxycholat |
| | 50,00 mmol/ | Bicine-Puffer |
| | 0,98 mg/l | Colipase |
| | 5,00 mmol/l | Calciumchlorid Konservierungsmittel |

| | | |
|---|---|---|
| Reagenz 2 (R2): | 8,1 mmol/l | Taurodesoxycholat |
| | 0.24 mmol/l | Farbsubstrat: z.B. 1,2-O-Dilauryl-rac-glycero-3-glutarsäure-(6'-methylresorufin)ester |
| | 1,60 mmol/l | Tartrat-Puffer |
| | 0,10 mmol/l | Calciumchlorid |
| | | Konservierungsmittel/Tenside |

Proben: NaCl mit verschiedenen Konzentrationen IgG (0 - 3 g/l)

Durchführung des Farbtestes am BM/Hitachi 717 Analysenautomaten gemäß folgender Applikation:
Temperature: 37°C

| PROGRAMM 2 CHEMISTRY PARAMTERS | |
|---|---|
| TEST | Lipase |
| ASSAY CODE (RATE A) | 5 - 32 - 39 |
| SAMPLE VOLUME (µl) | 7 - 3 |
| R 1 Volume (µl) | 250 - 100 - 0 |
| R 2 Volume (µl) | 50 - 50 - 0 |
| WAVELENGTH (nm) | 800 570 |
| CALIB. METHOD | 2 - 0 - 0 |
| STD. 1 CONC.-POS. | 0 - 1 |
| STD. 2 CONC.-POS. | assigned value - 2 |
| STD. 3 CONC.-POS. | 0 - 0 |
| STD. 4 CONC.-POS. | 0 - 0 |
| STD. 5 CONC.-POS. | 0 - 0 |
| STD. 6 CONC.-POS. | 0 - 0 |
| SD LIMIT | 0.1 |
| DUPLICATE LIMIT | 100 |
| SENSITIVITY LIMIT | 0 |
| ABS. LIMIT (INC/DEC) | 32000 - 0 |
| PROZONE LIMIT | 0 - 0 |
| EXPECTED VALUE (mg/dl) | 0 - 190 |
| PANIC VALUE (mg/dl) | ..... - ..... |
| INSTRUMENT FACTOR | 1 .0 |

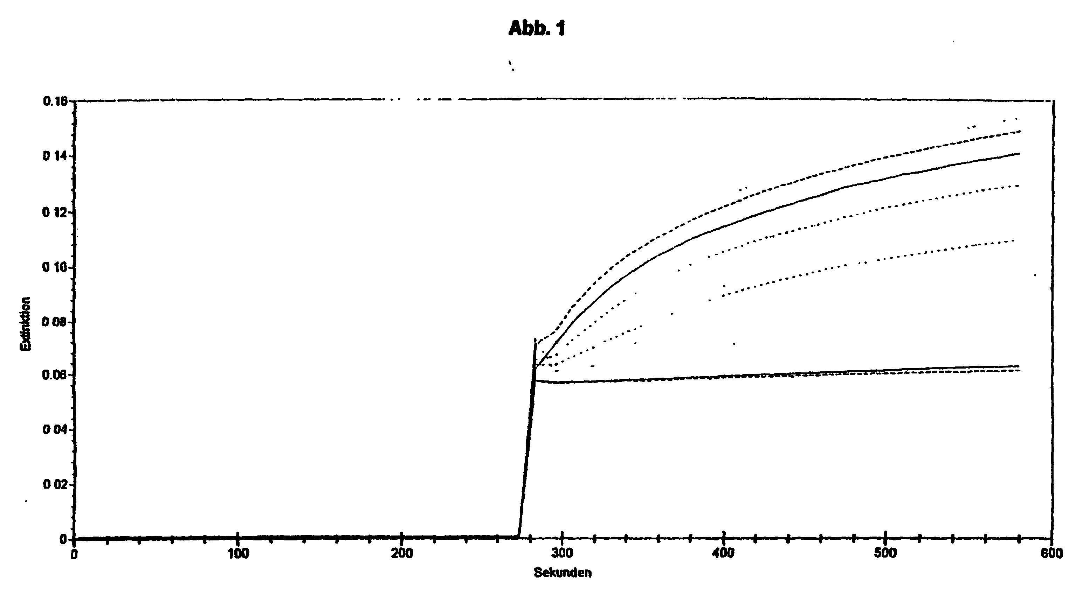

Auswertung: Durch Ablesung an einer Eichkurve, die mit Hilfe eines Nullstandards und eines Lipasestandards (Cfas) der Fa. Boehringer Mannheim ebenfalls unter den oben beschriebenen Gerätebedingungen ermittelt wurde.
Ergebnis: Es zeigte sich. daß mit einer eine erfindungsgemäße Substanz nicht enthaltenen Emulsion eine von der Konzentration des eingesetzten IgG's deutlich abhängige Hydrolyse des Farbsubstrats erfolgt (s. Abbildung 1).

### Beispiel 2

Verschiedene Konzentrationen des erfindungsgemäßen Zusatzes (Entstörmittels)

Die Reagenzzusammensetzung für den Lipase-Farbtest entspricht den in Beispiel 1 angegebenen Reagenzien R1 und R2 mit dem Unterschied, daß R1 zusätzlich 0.27 % eines Gemisches von Tetranatrium-N-(1,2-dicarboxyethyl)-N-akylsulfosuccinamid mit einem C12- bis C18-Alkylrest enthält.

Proben: NaCl mit verschiedenen Konzentrationen an IgG (0 - 3 g/l).

Die Durchführung und Auswertung des Farbtestes am BM/Hitachi 717 Analysen-automaten erfolgte gemäß der in Beispiel 1 beschriebenen Applikation bzw. Angaben.

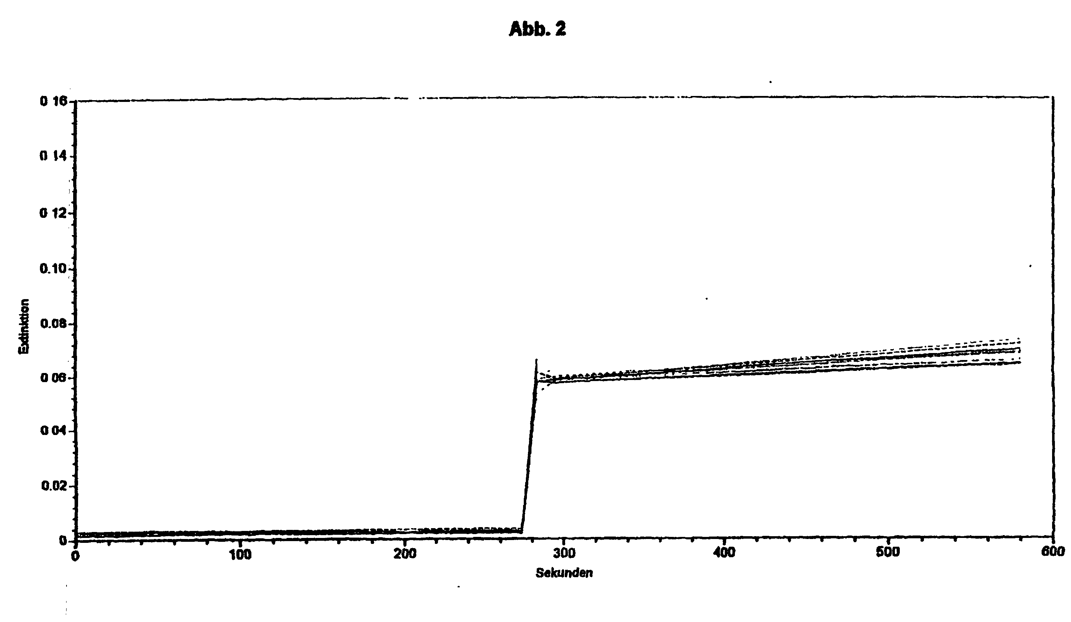

Ergebnis: Bei den vorgegebenen Konzentrationen wird eine unspezifische, nicht enzymatische Hydrolyse eliminiert (s. Abbildung 2).

### Beispiel 3

Herstellung des Gesamtreagenzes:
Reagenz 1: wie Beispiel 1
Reagenz 2:
   0,6 g Lipase-Farbsubstrat (z.B. 1,2-O-Dilauryl-rac-glycero-3-glutarsäure-(6'-methylresorufin)ester) wird in 9 ml eines geeigneten Alkohols, z.B. Ethanol, gelöst, Zu dieser Lösung wird 1 g eines Emulgators z.B. Brij 35 oder Triton X-114 gegeben. Diese Ölphase wird in einer Spritze aufgesogen und unter Druck durch eine sehr feine Kanüle (Innendurchmesser 0,15 bis 1.0 mm) in die, durch einen Rührer bewegte, Wasserphase gepreßt Die Wasserphase besteht z.B. aus 0.15 g Tartratpuffer (pH-Wert 5,0), 0,009 g Claciumchlorid sowie 0,5 g Taurodesoxycholat gelöst in 100 ml Wasser. In der Wasserphase können sich noch Konservierungsmittel sowie weitere Hilfsemulgatoren befinden.

### Beispiel 4

Vergleich turbidimetrische Methode/Farbtest ohne Zusatz erfindungsgemäßer Substanz

Die Reagenzzusammensetzung des Lipase-Farbtestes entspricht den in Beispiel 1 gemachten Angaben.

Die Reagenzzusammenstellung "Lipase turbidimetrischer Test" ist wie folgt:

| | | |
|---|---|---|
| R1: | 19,0 mmol/l | Na-Desoxycholat |
| | 26,0 mmol/l | Tris-Puffer |
| | 3,0 mg/ml | Colipase |
| | 0,1 mmol/l | Calciumchlorid |
| | 0,30 mmol/l | Triolein |
| | | Konservierungsmittel |

### Proben: Humanseren

Durchführung der verschiedenen Lipasebestimmungen am BM/Hitachi 717 Analysenautomaten gemäß folgender Applikation:

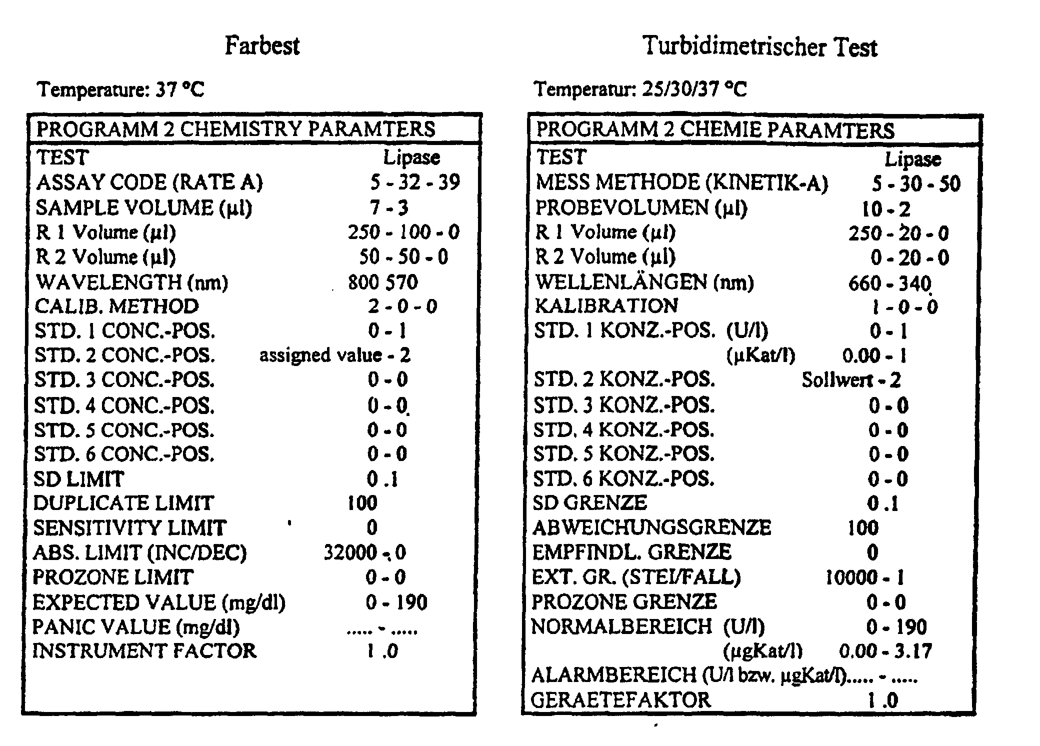

Die Auswertung erfolgte analog zu Beispiel 1.

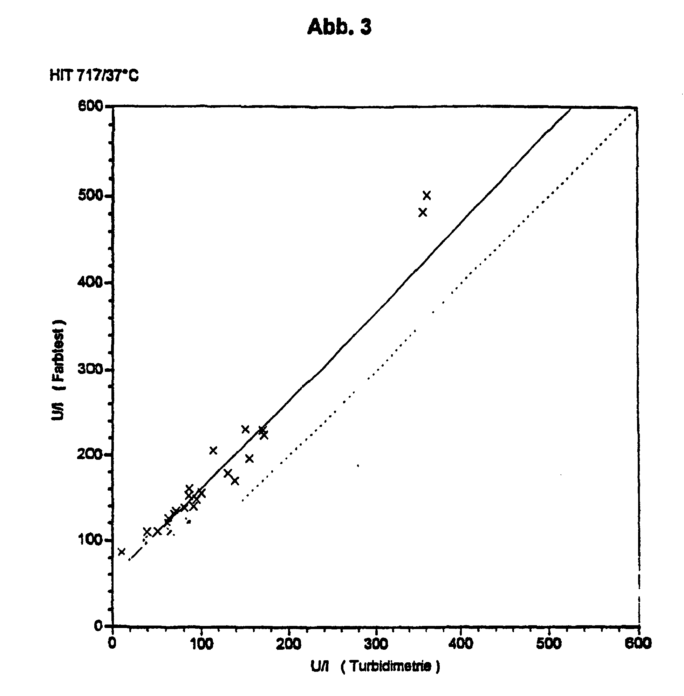

Ergebnis: Der Methodenvergleich zeigt, daß Farbteste einen Achsenabschnitt aufweisen, der auf der unspezifischen Hydrolyse des Farbsubstrates durch Serumbestandteile basiert (s. Abbildung 3).

### Beispiel 5

Vergleich turbidimetrische Methode/Farbtest mit Zusatz erfindungsgemäßer Substanz

Die Reagenzienzusammensetzung und Durchführung erfolgt wie unter Beispiel 4 beschrieben. Außerdem wurde als erfindungsgemäße Substanz Tetrasodium-N-(1,2-dicarboxyethyl)-N-octadecanyl-sulfosuccinimat oder ein entsprechendes die Verbindung enthaltendes Gemisch in einer Konzentration von 0,27 % (w/v) Reagenz 1 (R1) zugesetzt.

Proben: Humanseren
Die Durchführung der verschiedenen Lipasebestimmungen am BM/Hitachi 717 Analysenautomaten erfolgte gemäß der in Beispiel 4 angegebenen Applikation. Die Auswertung erfolgte ebenfalls analog zu Beispiel 4 bzw. 1.

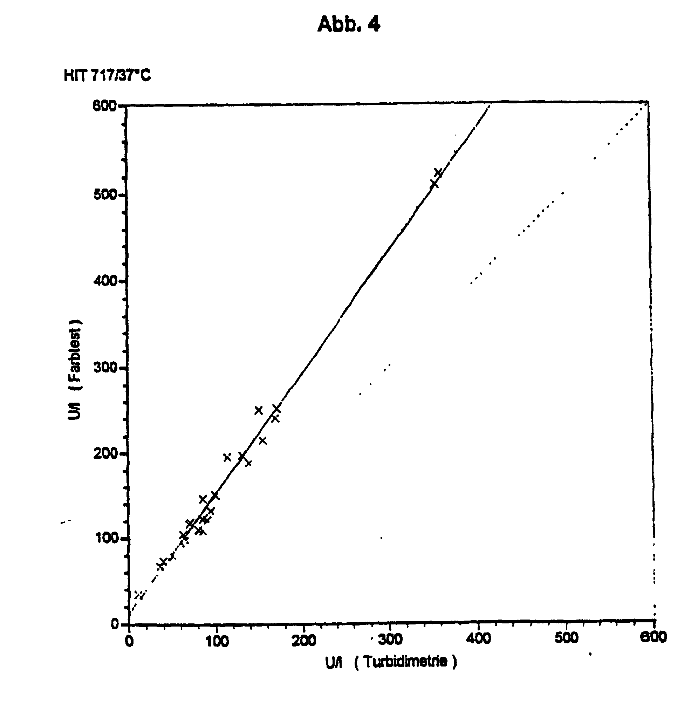

Ergebnis: Durch Zusatz der erfindungsgemäßen Substanz werden unspezifische Reaktionen eliminiert und somit der Achsenabschnitt im Vergleich zur turbidimetrischen Methode beseitigt (Abbildung 4).

## Patentansprüche

1. Verfahren zur Bestimmung von Lipase in einer biologischen Probe durch Zugabe eines Lipase-Farbsubstrates und anschließende photometrische Bestimmung des aus dem Substrat freigesetzten Farbstoffs, **dadurch gekennzeichnet, dass** die Bestimmung in Gegenwart eines N-substituierten Carbonsäureamid-Darivats mit einem Molekulargewicht bis zu 3000 Dalton durchgeführt wird.

2. Verfahren gemäß Anspruch 1, **dadurch gekennzeichnet, daß** die Bestimmung in Gegenwart einer Verbindung der allgemeinen Formel (I) bzw. (Ia) 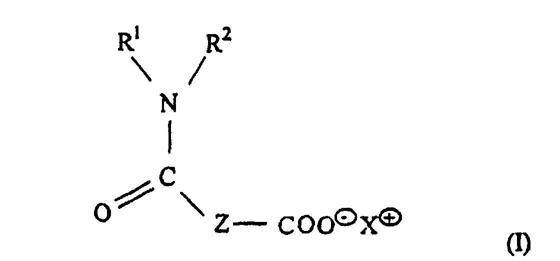 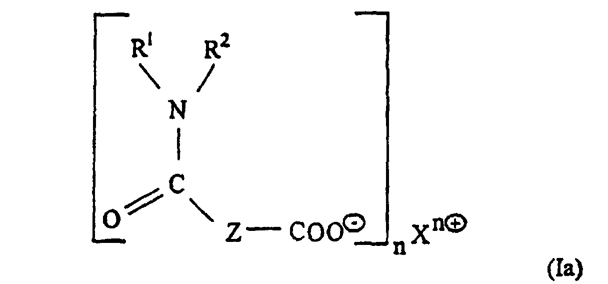 wobei R¹ und R², unabhängig voneinander, Wasserstoff oder einen gesättigten oder ungesättigten, substituierten oder unsubstituierten Kohlenwasserstoffrest mit 2 bis 24 Kohlenstoffatomen,
Z einen gesättigten oder ungesättigten, substituierten oder unsubstituierten, cyclischen oder geradkettigen Kohlenwasserstoffrest mit 1 bis 10 Kohlenstoffatomen,
X ein Atom bzw. eine Atomgruppe mit positiver Ladung und n eine Zahl von 1 bis 3 darstellen.

3. Verfahren nach Anspruch 2. **dadurch gekennzeichnet, daß** R¹ oder R² eine gegebenenfalls substituierte Alkyl-, Aryl-, Alkylaryl- oder Alkylengruppe bestehend aus 3 bis 24 C-Atomen.
Z eine Methylen- und/oder Ethylen-Gruppierung mit bis 10 C-Atome aufweist und gegebenenfalls durch eine oder mehrere Carboxyl-, Sulfonyl-. Phosphat-, Phosphonat-, Nitro-, Nitrit- oder Nitrat-, Halogen- oder Alkoxygruppe substituiert ist.

4. Verfahren nach einem der Ansprüche 2 oder 3, **dadurch gekennzeichnet, daß** eine Verbindung bzw. ein Salz der allgemeinen Formel (I) oder (Ia) zugesetzt wird, wobei der Rest Z eine Carboxyl- oder Sulfonylgruppe trägt.

5. Verfahren nach einem der Ansprüche 2 bis 4. **dadurch gekennzeichnet, daß** einer der Reste R¹ oder R² eine Methyl-, Ethyl-, Propyl-, Butyl-, Pentyl-, Ethylenyl- oder eine Propylenylgruppe oder eine C12- bis C18-Alkylgruppe bedeutet.

6. Verfahren nach einem der Ansprüche 2 bis 5. **dadurch gekennzeichnet. daß** als Salz ein Tetranatrium-N-(1,2-dicarboxylethyl)-N-alkyl-sulfosuccinamid. Tetranatrium-N-(1,2-dicarboxylethyl)-N-alkylaryl-sulfosuccinamid oder ein davon abgeleitetes Derivat bzw. ein entsprechendes. eine solche Verbindung enthaltenes Gemisch ist.

7. Verfahren nach einem der Ansprüche 1 bis 6. **dadurch gekennzeichnet, daß** das Carbonsäureamid-Derivat in einer Konzentration von 0.001 bis 2.0% (w/v) in der Probenlösung vorliegt.

8. Verfahren nach einem der vorangegangenen Ansprüche, **dadurch gekennzeichnet, daß** die Verbindung bzw. entsprechende Verbindungen in einer Endkonzentration von 0.01 bis 1,0% (w/v) vorliegen.

9. Verfahren gemäß einem der vorangegangenen Ansprüche, **dadurch gekennzeichnet, daß** humane Pankreaslipase oder Pankreaslipase vom Tier bestimmt wird.

10. Reagenz zur Bestimmung von Lipase enthaltend folgende Komponenten:
(a) ein Lipase-Substrat
(b) eine Puffersubstanz und
(c) ein N-substituiertes Carbonsäureamid-Derivat mit einem Molekulargewicht bis zu 3000 Dalton.

11. Reagenz gemäß Anspruch 10 enthaltend folgende Komponenten:
(a) ein Lipase-Farbsubstrat.
(b) eine Puffersubstanz.
(c) eine Verbindung der allgemeinen Formel (I) oder (Ia) 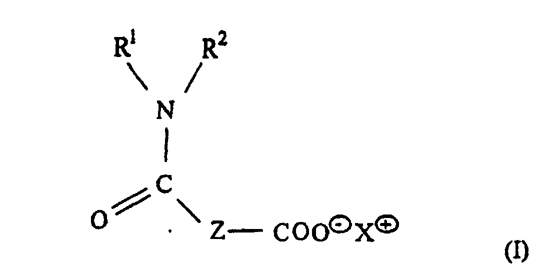 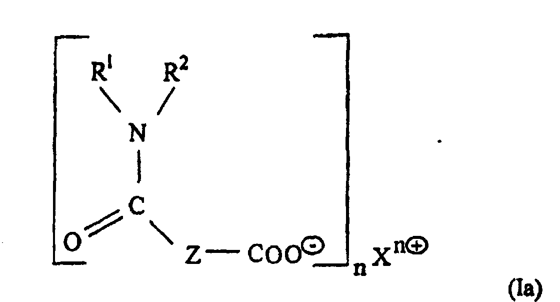
wobei R¹ und R², unabhängig voneinander, Wasserstoff oder einen gesättigten oder ungesättigten, substituierten oder unsubstituierten Kohlenwasserstoffrest mit 2 bis 24 Kohlenstoffatomen,
Z einen gesättigten oder ungesättigten, substituierten oder unsubstituierten, cyclischen oder geradkettigen Kohlenwasserstoffrest mit 1 bis 10 Kohlenstoffatomen,
X ein Atom bzw. eine Atomgruppe mit positiver Ladung und n eine Zahl von 1 bis 3 darstellen.

12. Reagenz nach Anspruch 10 oder 11, **dadurch gekennzeichnet, daß** das Reagenz in einem ersten Teilreagenz eine Puffersubstanz (b) mit einem pH-Wert von ca. 7,5 bis 9,0, eine Komponente (c), ein Detergenz und/oder Konservierungsmittel enthält und das zweite Teilreagenz eine Puffersubstanz (b) mit einem pH-Wert von ca. 2,0 bis 5,0, eine Komponente (a) und einen Emulgator enthält.

13. Reagenz nach einem der Ansprüche 10, 11 oder 12, **dadurch gekennzeichnet, daß** es sich bei Komponente (c) um ein Tetranatrium-N-(1.2-dicarboxyethyl)-N-alkyl-sulfosuccinamid. Tetranatrium-N-(1,2-dicarboxyethyl)-N-alkylaryl-sulfosuccinamid oder ein entsprechendes Derivat bzw. um ein entsprechendes, eine solche Verbindung enthaltenes Gemisch handelt.

14. Verwendung eines N-substituierten Carbonsäureamid-Derivats bzw, einer Verbindung der allgemeinen Formel (I) oder (Ia) 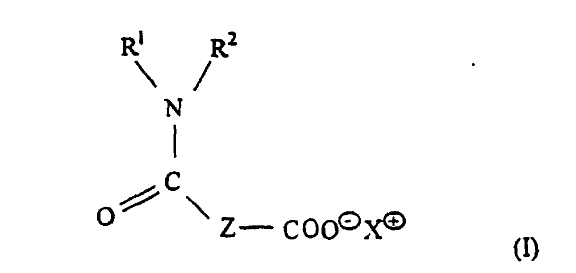 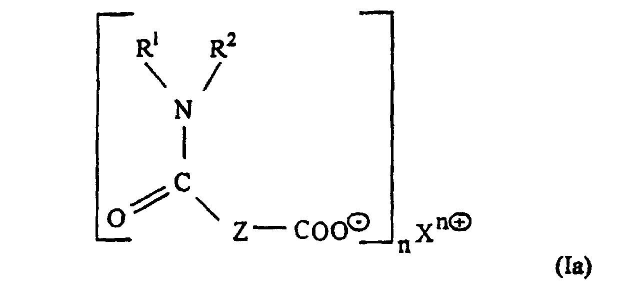 wobei R¹ und R², unabhängig voneinander. Wasserstoff oder einen gesättigten oder ungesättigten, substituierten oder unsubstituierten Kohlenwasserstoffrest mit 2 bis 24 Kohlenstoffatomen.
Z einen gesättigten oder ungesättigten, substituierten oder unsubstituierten, cyclischen oder geradkettigen Kohlenwasserstoffrest mit 1 bis 10 Kohlenstoffatomen,
X ein Atom bzw, eine Atomgruppe mit positiver Ladung und n eine Zahl von 1 bis 3 darstellen,
zur Eliminierung von unspezifischen Reaktionen bei der Bestimmung von Lipase in biologischem Probenmaterial.

## Claims

1. Method for the determination of lipase in a biological sample by adding a lipase colour substrate and subsequently photometrically determining the dye released from the substrate, wherein the determination is carried out in the presence of an N-substituted carboxylic acid amide derivative, having a molecular weight of up to 3000 Dalton.

2. Method as claimed in claim 1, wherein the determination is carried out in the presence of a compound of the general formula (I) or (Ia) 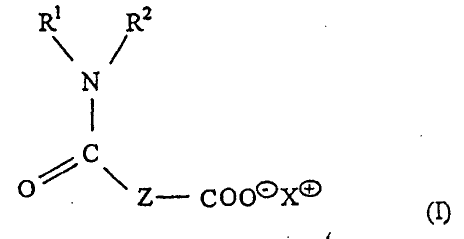 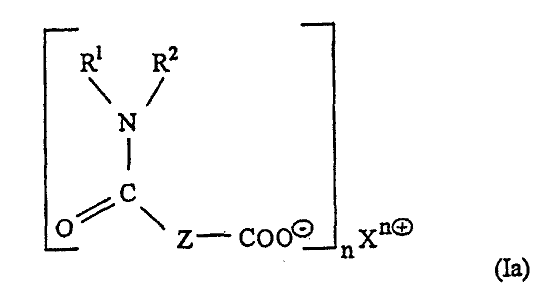 in which R¹ and R² independently of one another represent hydrogen or a saturated or unsaturated, substituted or unsubstituted hydrocarbon residue with 2 to 24 carbon atoms,
Z denotes a saturated or unsaturated, substituted or unsubstituted, cyclic or straight-chained hydrocarbon residue with 1 to 10 carbon atoms,
X represents an atom or a group of atoms with positive charge and n is a number from 1 to 3.

3. Method as claimed in claim 2, wherein R¹ or R² represent an optionally substituted alkyl, aryl, alkylaryl or alkylene group composed of three to 24 C atoms,
Z has a methylene and/or ethylene group with up to 10 C atoms and is optionally substituted by one or several carboxyl, sulfonyl, phosphate, phosphonate, nitro, nitrite or nitrate, halogen or alkoxy groups.

4. Method as claimed in one of the claims 2 or 3, wherein a compound or a salt of the general formula (I) or (Ia) is added in which Z carries a carboxyl or sulfonyl group.

5. Method as claimed in one of the claims 2 to 4, wherein one of the residues R¹ or R² denotes a methyl, ethyl, propyl, butyl, pentyl, ethylenyl or a propylenyl group or a C12 to C18 alkyl group.

6. Method as claimed in one of the claims 2 to 5, wherein the salt is a tetrasodium-N-(1,2-dicarboxylethyl)-N-alkyl-sulfosuccinamide, tetrasodium-N-(1,2-dicarboxylethyl)-N-alkylaryl-sulfosuccinamide or a derivative derived therefrom or a corresponding mixture containing such a compound.

7. Method as claimed in one of the claims 1 to 6, wherein the carboxylic acid amide derivative is present in the sample solution at a concentration of 0.001 to 2.0 % (w/v).

8. Method as claimed in one of the previous claims, wherein the compound or corresponding compounds are present at a final concentration of 0.01 to 1.0% (w/v).

9. Method as claimed in one of the previous claims, wherein human pancreatic lipase or pancreatic lipase from an animal is determined.

10. Reagent for the determination of lipase containing the following components:
(a) a lipase substrate,
(b) a buffer substance and
(c) an N-substituted carboxylic acid amide derivative having a molecular weight of up to 3000 Dalton.

11. Reagent as claimed in claim 10 containing the following components:
(a) a lipase colour substrate,
(b) a buffer substance,
(c) a compound of the general formula (I) or (Ia) 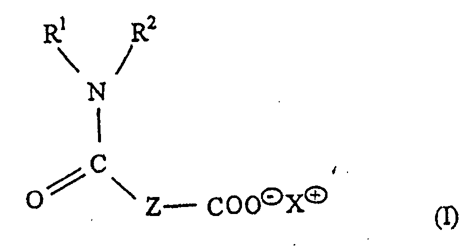 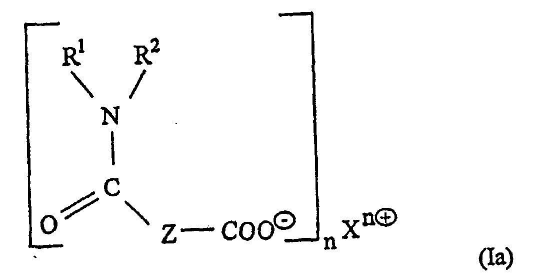 in which R¹ and R² independently of one another represent hydrogen or a saturated or unsaturated, substituted or unsubstituted hydrocarbon residue with 2 to 24 carbon atoms
Z represents a saturated or unsaturated, substituted or unsubstituted, cyclic or straight-chained hydrocarbon residue with 1 to 10 carbon atoms,
X represents an atom or a group of atoms with a positive charge and n is a number from 1 to 3.

12. Reagent as claimed in claim 10 or 11, wherein the reagent in a first partial reagent contains a buffer substance (b) with a pH value of ca. 7.5 to 8.9, a component (c), a detergent and/or preservative and the second partial reagent contains a buffer substance (b) with a pH value of ca. 2.0 to 5.0, a component (a) and an emulsifier.

13. Reagent as claimed in one of the claims 10, 11 or 12, wherein component (c) is a tetrasodium-N-(1,2-dicarboxyethyl)-N-alkylsulfosuccinamide, tetrasodium-N-(1,2-dicarboxyethyl)-N-alkylaryl-sulfosuccinamide or a corresponding derivative or an appropriate mixture containing such a compound.

14. Use of an N-substituted carboxylic acid amide derivative or a compound of the general formula (I) or (Ia) 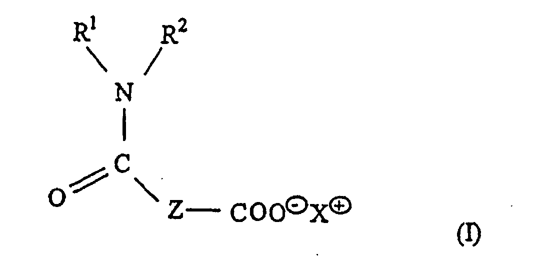 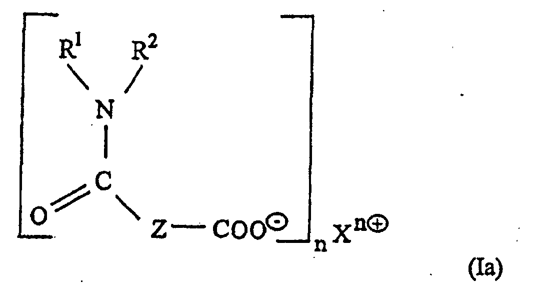 in which R¹ and R² independently of one another represent hydrogen or a saturated or unsaturated, substituted or unsubstituted hydrocarbon residue with 2 to 24 carbon atoms,
Z denotes a saturated or unsaturated, substituted or unsubstituted, cyclic or straight-chained hydrocarbon residue with 1 to 10 carbon atoms,
X represents an atom or a group of atoms with positive charge and n is a number from 1 to 3,
for the elimination of unspecific reactions in the determination of lipase in biological sample material.

## Revendications

1. Procédé pour la détermination de la teneur en lipase d'un échantillon biologique par addition d'un substrat coloré de lipase et par détermination photométrique ultérieure du colorant libéré du substrat, **caractérisé en ce qu'**on effectue la détermination en présence d'un dérivé d'amide d'acide carboxylique substitué sur l'atome d'azote, dont le poids moléculaire s'élève jusqu'à 3000 Daltons.

2. Procédé selon la revendication 1, **caractérisé en ce que** la détermination a lieu en présence d'un composé répondant à la formule générale (I), respectivement (la) 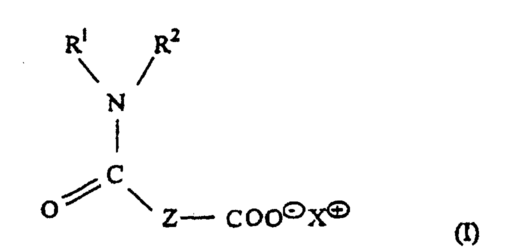 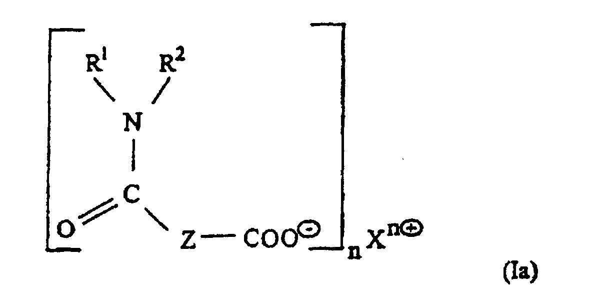 dans lesquelles R¹ et R² représentent, indépendamment l'un de l'autre, un atome d'hydrogène ou un radical d'hydrocarbure saturé ou insaturé, substitué ou non substitué, contenant de 2 à 24 atomes de carbone,
Z représente un radical d'hydrocarbure saturé ou non saturé, substitué ou non substitué, cyclique ou à chaîne droite, contenant de 1 à 10 atomes de carbone,
X représente un atome, respectivement un groupe d'atomes possédant une charge positive et n représente un nombre de 1 à 3.

3. Procédé selon la revendication 2, **caractérisé en ce que** R¹ ou R² représente un groupe alkyle, un groupe aryle, un groupe alkylaryle ou un groupe alkylène, le cas échéant substitué, constitué par un nombre de 3 à 24 atomes de carbone,
Z représente un groupement méthylène et/ou un groupement éthylène contenant jusqu'à 10 atomes de carbone et est substitué, le cas échéant, par un ou plusieurs groupes carboxyle, sulfonyle, phosphate, phosphonate, nitro, nitrite ou nitrate, par un ou plusieurs atomes d'halogène ou par un ou plusieurs groupes alcoxy.

4. Procédé selon l'une quelconque des revendications 2 ou 3, **caractérisé en ce qu'**on ajoute un composé, respectivement un sel répondant à la formule générale (I) ou (la), le radical Z portant un groupe carboxyle ou un groupe sulfonyle.

5. Procédé selon l'une quelconque des revendications 2 à 4, **caractérisé en**
**ce qu'**un des radicaux R¹ ou R² représente un groupe méthyle, un groupe éthyle, un groupe propyle, un groupe butyle, un groupe pentyle, un groupe éthylényle ou un groupe propylényle ou encore un groupe alkyle en C12-C18.

6. Procédé selon l'une quelconque des revendications 2 à 5, **caractérisé en ce qu'**on utilise, à titre de sel, un N-(1,2-dicarboxyéthyl)-N-alkyl-sulfosuccinamide tétrasodique, un N-(1,2-dicarboxyéthyl)-N-alkylaryl-sulfosuccinamide tétrasodique ou un de leurs dérivés, respectivement un mélange correspondant contenant un tel composé.

7. Procédé selon l'une quelconque des revendications 1 à 6, **caractérisé en ce que** le dérivé d'amide d'acide carboxylique est présent en une concentration de 0,001 à 2,0 % (en poids/volume) dans la solution d'échantillon.

8. Procédé selon l'une quelconque des revendications précédentes, **caractérisé en ce que** le composé, respectivement les composés correspondants sont présents en une concentration finale de 0,01 à 1,0 % (en poids/volume).

9. Procédé selon l'une quelconque des revendications précédentes, **caractérisé en ce que** l'on détermine la teneur de la lipase pancréatique humaine ou de la lipase pancréatique d'un animal.

10. Réactif pour la détermination de la teneur en lipase contenant les composants ci-après :
(a) un substrat de lipase,
(b) une substance tampon, et
(c) un dérivé d'amide d'acide carboxylique substitué sur l'atome d'azote, dont le poids moléculaire s'élève jusqu'à 3000 Daltons.

11. Réactif selon la revendication 10 contenant les composants ci-après :
(a) un substrat coloré de lipase,
(b) une substance tampon, et
(c) un composé répondant à la formule générale (I) ou (la) 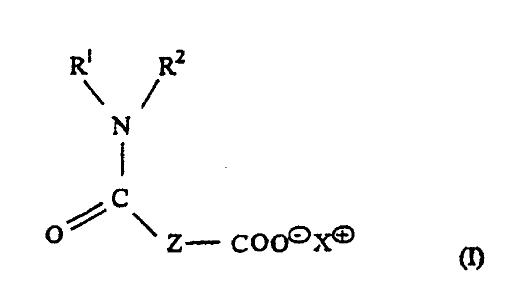 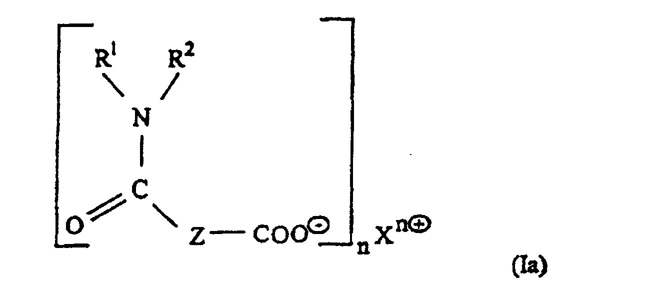
dans lesquelles R¹ et R² représentent, indépendamment l'un de l'autre, un atome d'hydrogène ou un radical d'hydrocarbure saturé ou insaturé, substitué ou non substitué, contenant de 2 à 24 atomes de carbone,
Z représente un radical d'hydrocarbure saturé ou non saturé, substitué ou non substitué, cyclique ou à chaîne droite, contenant de 1 à 10 atomes de carbone,
X représente un atome, respectivement un groupe d'atomes possédant une charge positive et n représente un nombre de 1 à 3.

12. Réactif selon la revendication 10 ou 11, **caractérisé en ce que** le réactif contient, dans un premier réactif partiel, une substance tampon (b) possédant une valeur de pH d'environ 7,5 à 9,0, un composant (c), un détergent et/ou un agent conservateur, le deuxième réactif partiel contenant une substance tampon (b) possédant une valeur de pH d'environ 2,0 à 5,0, un composant (a) et un émulsifiant.

13. Réactif selon l'une quelconque des revendications 10, 11 ou 12, **caractérisé en ce qu'**il s'agit, en ce qui concerne le composant (c), d'un N-(1,2-dicarboxyéthyl)-N-alkyl-sulfo-succinamide tétrasodique, d'un N-(1,2-dicarboxyéthyl)-N-alkylaryl-sulfo-succinamide tétrasodique ou d'un dérivé correspondant, respectivement d'un mélange correspondant contenant un tel composé.

14. Utilisation d'un dérivé d'amide d'acide carboxylique substitué sur l'atome d'azote, respectivement d'un composé répondant à la formule générale (I) ou (la) 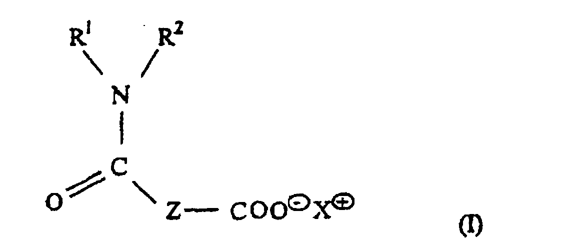 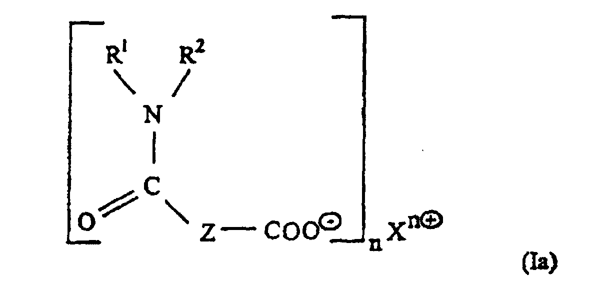 dans lesquelles R¹ et R² représentent, indépendamment l'un de l'autre, un atome d'hydrogène ou un radical d'hydrocarbure saturé ou insaturé, substitué ou non substitué, contenant de 2 à 24 atomes de carbone,
Z représente un radical d'hydrocarbure saturé ou non saturé, substitué ou non substitué, cyclique ou à chaîne droite, contenant de 1 à 10 atomes de carbone,
X représente un atome, respectivement un groupe d'atomes possédant une charge positive et n représente un nombre de 1 à 3.
pour l'élimination de réactions non spécifiques lors de la détermination de la teneur en lipase d'une matière d'échantillon biologique.
